# EUROPEAN PATENT APPLICATION

(11) **EP 2 494 978 A1**
(43) Date of publication of application: **05.09.2012**
(21) Application number: 11156842.4
(22) Date of filing: 03.03.2011
(51) Int. Cl.: A61K 35/76, C12N 7/04, C07K 14/705

(54) **Enhanced tumor therapy by tumor stem cell targeted oncolytic viruses**

(71) Applicant: Bundesrepublik Deutschland, Letztvertreten Durch Den Präsidenten Des Paul-Ehrlich-Instituts, 63225 Langen (DE)
(72) Inventor: Buchholz, Christian, 60594 Frankfurt (DE); Abel, Tobias, 63225 Langen (DE); Bach, Patricia, 63512 Hainburg (DE)
(74) Representative: Grund, Martin

(57) **Abstract**

The invention relates to recombinant oncolytic viruses that target tumor stem cells and various uses of these recombinant viruses. In particular, an oncolytic virus comprising a recombinant binding domain specific for a tumor stem cell marker is disclosed. Furthermore, the use of such oncolytic viruses for the treatment of cancer is disclosed.

## Description

### FIELD OF THE INVENTION

The invention relates to recombinant oncolytic viruses that target tumor stem cells and various uses of these recombinant viruses.

### BACKGROUND OF THE INVENTION

Tumors are thought to comprise a heterogeneous tumor cell population that differs in the degree of differentiation of the cells. A small fraction of the tumor cell population of a given tumor is made up of so-called tumor stem cells. It was shown that such tumor stem cells have a more pronounced ability to spread and/or generate new tumors than most of the further differentiated tumor cells of a given tumor. Such tumor stem cells can be identified by their surface markers, e.g. CD133.

The glycoprotein CD133 is primarily expressed on undifferentiated cells such as stem cells and precursor cells. The expression of CD133 has been shown for various stem cells such as hematopoietic stem cells, endothelial stem cells and neural stem cells and various tumors.

The fraction of CD133 positive tumor cells within a given tumor is quite low, however these cells are the prime source for new tumors, resistant to chemotherapy and feature a high regenerative potential.

Therefore, many types of cancer are still not curable and a need for new therapies exists.

### SUMMARY OF THE INVENTION

The present invention is based on the surprising finding that recombinant oncolytic viruses comprising a binding domain specific for a cell marker for a tumor stem cell can be utilized to specifically target/attack tumor stem cells comprised by a tumor, thereby inhibiting further growth and/or metastasizing of the tumor. Even more surprisingly, it has been found that such recombinantly modified oncolytic viruses exhibit a significantly increased oncolytic potential as compared to their non-modified original counterparts.

The present invention thus provides an improved treatment of various types of cancer, and even such types that have as of yet been thought to be not curable, is possible.

In one embodiment, the invention is directed to an oncolytic virus comprising a recombinant **binding** domain specific for a tumor stem cell marker.

The term **oncolytic virus** is meant to comprise any virus that infects/enters and lyses cancer cells. The ideal oncolytic virus efficiently kills a clinically relevant fraction of the patient cancer cells by direct cytolysis with a minimal destruction of non-neoplastic tissue. Targeted tumor cell entry and specificity of replication are desirable. Furthermore, the virus should be safe and apathogenic when applied in patients. Oncolytic viruses derived from many different types of viruses have been described by Liu et al. (Liu et al., Nature Clinical Practice Oncology 4: (2) 101-117, 2007). Among these enveloped viruses such as herpes simplex virus (HSV), vaccinia virus (VV) and paramyxoviruses such as measles virus (MeV), Newcastle disease virus (NDV) or rhabdoviruses like vesicular stomatitis virus (VSV), are most prominent. Besides applying unmodified wildtype virus, genetic engineering can further improve safety and efficacy of oncolytic viruses. Engineering the envelope proteins can restrict virus infection to tumor cells and insertion of suicide genes can enhance therapeutic effects (Nakamura et al., Expert Opin. Bio. Ther. 4: (10): 1685-1692, 2004); Liu et al., Nature Clinical Practice Oncology 4: (2) 101-117, 2007).

In preferred embodiments the oncolytic virus is an enveloped virus derived from the virus families herpesviridae, poxviridae, rhabdoviridae, or paramyxoviridae, preferably from the *Paramyxoviridae* family, genus Morbillivirus, more preferably a MeV or a vaccine strain of MeV such as the Edmonston strain (MeV_{Edm}). MeV utilizes two envelope glycoproteins (the fusion protein (F) and the hemagglutinin protein (H)) to gain entry into the target cell. Protein F is a type I transmembrane protein, while protein H is a type II transmembrane domain, i.e. its amino-terminus is exposed directly to the cytoplasmic region. Both proteins thus comprise a transmembrane and a cytoplasmic region. One known function of the F protein is mediating the fusion of viral membranes with the cellular membranes of the host cell. Functions attributed to the H protein include recognizing the receptor on the target membrane and supporting F protein in its membrane fusion function. The direct and highly efficient membrane fusion at the cellular surface membrane is a particular property of measles virus and the morbilliviruses, thus distinguishing themselves from many other enveloped viruses that become endocytosed and will only fuse upon pH drop upon endocytosis. Both proteins are organized on the viral surface in a regular array of tightly packed spikes, H tetramers, and F trimers (Russell et al., Virology 199:160-168,1994).

The Edmonston strain of MeV (MeV_{Edm}) uses a single protein as its main receptor, namely, the protein known to be the regulator of complement activation factor, CD46 (Gerlier et al., Trends Microbiol. 3:338-345, 1995). CD46 is expressed on all nucleated human cells. Most clinical isolates of measles virus, however, cannot effectively use CD46 as a receptor. Human SLAM (signaling lymphocyte-activation molecule; also known as CDw150) is a recently discovered membrane glycoprotein that is expressed on some T and B cells, and was also found to act as a cellular receptor for MeV, including the Edmonston strain (Tatsuo et al., Nature 406(6798):893-7, 2000). The precise biological functions and interactions of the MeV H and F proteins remain largely unclear.

The term **recombinant binding domain** is meant to comprise any domain of the virus that allows the virus to gain entry into the target cell and that is not present in the naturally occurring virus. Preferably, the binding domain is a protein or part of a protein, more preferably the binding domain is an envelope protein of the virus or comprised by an envelope protein of the virus. Even more preferably the binding domain is a ligand to a receptor present on the target cell. Still more preferably, the binding domain is an antibody, antibody fragment and or a single-chain variable fragment (scFv). Most preferably, the binding domain is an scFv and/or specific for the tumor stem cell marker CD133.

The term **specific for a tumor stem cell marker** is to be understood as the ability of the recombinant binding domain to interact with the tumor stem cell marker. Preferably, this interaction is a binding of the binding domain to said marker. More preferably, this binding is brought about by protein-protein interactions. Even more preferably, the binding domain acts as a ligand, while the tumor stem cell marker acts as a receptor. The concept of ligand and receptor interactions is well known to the skilled person. The term **cell marker** as used in the present invention, refers to a molecule present on the surface of a cell. Such molecules can be, inter alia, peptides or proteins that may comprise sugar chains or lipids, antigens, clusters of differentiation (CDs), antigens, antibodies or receptors. Since not all populations of cells express the same cell markers, a cell marker can thus be used to identify, select or isolate a given population of cells expressing a specific cell marker. As an example, CD4 is a cell marker expressed by T helper cells, regulatory T cells, and dendritic cells. Thus, T helper cells, regulatory T cells, and dendritic cells can be identified, selected or otherwise isolated, inter alia by a FACS cell sorter, by means of the CD4 cell marker. Likewise, CD133 is expressed on the surface of tumor stem cells. Preferably, the cell marker is a tumor stem cell marker, i.e. specific for tumor stem cells. Even more preferably, the tumor stem cell marker is CD133.

In a preferred embodiment of the invention the oncolytic virus has a decreased specificity for its original receptor(s) used for cell entry. In this embodiment the oncolytic virus of the present invention is preferably further modified in that the ligand(s) which are naturally used by the virus to gain entry to its host cell have a decreased specificity for their receptor(s). Preferably, the ligand(s) which are naturally used by the virus to gain entry to its host cell are modified to have no specificity or substantially no specificity for their receptor(s). Such a modification is preferably brought about by a mutation of the original ligands, more preferably by at least one point mutation within the ligand(s). The person skilled in the art will readily be able to introduce mutations as, for example, additions and deletions, into a given nucleic acid or amino acid sequence. Such known methodologies are, for example, disclosed in Sambrook et al. (1989). In a especially preferred embodiment in which the oncolytic virus is MeV, at least one point mutation is introduced into the H protein used for cell entry.

Mutation of the MeV H protein generally ablates productive interactions with CD46 and SLAM, respectively. In one embodiment, this mutation is introduced by the point mutations Y481A and R533A of the MeV H protein. In another embodiment, the Hmut protein also includes the mutations S548L and/or F549S, which lead to a more complete ablation of residual infectivity via CD46. Also, the mutation of the residues V451 and Y529 ablates productive interaction with CD46 and SLAM. Alternative mutations for ablating/preventing interaction of the H protein with CD46 have been described above. All of these mutations, which are introduced into the H proteins in order to ablate the natural receptor usage, are located in the ectodomain of the MeV H protein. For preventing interaction of the H protein with SLAM one or more of the following residues may be replaced with any other amino acid, in particular, alanine: I194, D530, Y553, T531, P554, F552, D505, D507 (See: Vongpunsawad et al. (2004) J Virol 78 (1) p. 302-313); Masse et al. (2004) J Virol 78 (17) p. 9051-9063).

In a further preferred embodiment of the invention the recombinant binding domain of the oncolytic virus is specific for a tumor stem cell marker expressed at the cell surface, such as CD44 (colon carcinoma, breast cancer) or CD133 (glioma, pancreatic adenocarcinoma, etc.). The CD133 cell marker is a five transmembrane domain glycoprotein (5-TM) that was initially shown to be expressed on primitive cell populations, including CD34 hematopoietic stem and progenitor cells, and other primitive cells such as retina and retinoblastoma and developing epithelium. The CD133 antigen belongs to a newly characterized molecular family of 5-TM proteins. No natural ligand has yet been demonstrated for the CD133 molecule, and its precise function in hematopoietic tissue remains unknown.

More preferably the recombinant binding domain comprises an antibody, a fragment of an antibody, or, most preferably a scFv. Even more preferably, said antibody, fragment of an antibody, or scFv is specific for CD133. In the most preferred embodiment the binding domain comprises an scFv derived from the hybridoma cell line deposited at ATCC as HB-12346. The generation of an scFv from a hybridoma cell line is a technique known to the skilled person and is exemplified in the appended examples (see Example 1).

In a further embodiment of the present invention the oncolytic virus further comprises at least one suicide gene. A suicide gene is a concept known to the skilled person. Generally it will, upon expression, cause a cell to be killed, preferably through apoptosis. In a further embodiment a suicide gene is used to make a tumor cell more sensitive to chemotherapy. Such an approach preferably involve a suicide gene that is coding for a toxic metabolit and/or an enzyme not found in the host of the tumor cell that can convert a harmless substance (pro-drug) into a toxic metabolite. A preferable suicide-transgene encodes for SuperCD, a fusion protein composed of yeast cytosine-deaminase and uracil-ribosyltranferase. When expressed from an oncolytic virus in tumor cells, it will convert the nontoxic prodrug 5-fluorcytosin, into the highly toxic compound 5-fluorouracil (Graepler et al., World J. Gastroenterol. 11: 6910-6919, 2005).

In another aspect the present invention is directed to the inventive oncolytic virus as a medicament. The oncolytic virus according to the invention is preferably comprised by a pharmaceutic composition.

Pharmaceutical compositions based on the oncolytic viruses of the present invention can be formulated in any conventional manner using one or more physiologically acceptable carriers or excipients. Thus, the oncolytic viruses of the present invention may be formulated for administration by, for example, injection, inhalation or insulation (either through the mouth or the nose) or by oral, buccal, parenteral or rectal administration.

The pharmaceutical compositions of the present invention can be formulated for a variety of modes of administration, including systemic, topical or localized administration. Techniques and formulations can be found in, for example, Remrnington's Pharmaceutical Sciences, Meade Publishing Co., Easton, Pa. For systemic administration, injection is preferred, including intramuscular, intravenous, intraperitoneal, and subcutaneous. For the purposes of injection, the pharmaceutical compositions of the present invention can be formulated in liquid solutions, preferably in physiologically compatible buffers, such as Hank's solution or Ringer's solution. In addition, the pharmaceutical compositions may be formulated in solid form and redissolved or suspended immediately prior to use. Lyophilized forms of the pharmaceutical composition are also suitable.

For oral administration, the pharmaceutical compositions of the present invention may take the form of, for example, tablets or capsules prepared by conventional means with pharmaceutically acceptable excipients such as binding agents (e.g. pregelatinized maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulfate). The tablets can also be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. ationd oil, oily esters, ethyl alcohol or fractionated vegetable oils); and preservatives (e.g. methyl or propyl-p-hydroxybenzoates or sorbic acid). The preparations can also contain buffer salts, flavoring, coloring and sweetening agents as appropriate.

The pharmaceutical compositions can be formulated for parenteral administration by injection, e.g. by bolus injection or continuous infusion. Formulations for injection can be presented in a unit dosage form, e.g. in ampoules or in multi-dose containers, with an optionally added preservative. The pharmaceutical compositions can further be formulated as suspensions, solutions or emulsions in oily or aqueous vehicles, and may contain other agents including suspending, stabilizing and/or dispersing agents.

Additionally, the pharmaceutical compositions can also be formulated as a depot preparation. These long acting formulations can be administered by implantation (e.g. subcutaneously or intramuscularly) or by intramuscular injection. Thus, for example, the compounds may be formulated with suitable polymeric or hydrophobic materials (e.g. as an emulsion in an acceptable oil) or ion exchange resins, or as sparingly soluble derivatives, for example, as a sparingly soluble salt. Other suitable delivery systems include microspheres, which offer the possibility of local noninvasive delivery of drugs over an extended period of time. This technology can include microspheres having a precapillary size, which can be injected via a coronary catheter into any selected part of an organ without causing inflammation or ischemia. The administered therapeutic is then slowly released from the microspheres and absorbed by the surrounding cells present in the selected tissue.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts, and fusidic acid derivatives. In addition, detergents may be used to facilitate permeation. Transmucosal administration can occur using nasal sprays or suppositories. For topical administration, the vector particles of the invention can be formulated into ointments, salves, gels, or creams as generally known in the art. A wash solution can also be used locally to treat an injury or inflammation in order to accelerate healing.

In a further embodiment the invention is directed to the oncolytic virus of the invention for the treatment or prevention of cancer, including, but not limited to, neoplasms, tumors, metastases, or any disease or disorder characterized by uncontrolled cell growth, and particularly multidrug resistant forms thereof. Examples of types of cancer and proliferative disorders to be treated with the therapeutics of the invention include, but are not limited to, leukemia (e.g. myeloblastic, promyelocytic, myelomonocytic, monocytic, erythroleukemia, chronic myelocytic (granulocytic) leukemia, and chronic lymphocytic leukemia), lymphoma (e.g. Hodgkin's disease and non-Hodgkin's disease), fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilms' tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia. In a particular embodiment, therapeutic compounds of the invention are administered to patients having prostate cancer (e.g., prostatitis, benign prostatic hypertrophy, benign prostatic hyperplasia (BPH), prostatic paraganglioma, prostate adenocarcinoma, prostatic intraepithelial neoplasia, prostato-rectal fistulas, and atypical prostatic stromal lesions). In a especially preferred embodiment the medicaments of the present invention are used for the treatment of cancer, glioma, liver carcinoma and/or colon carcinoma. The treatment and/or prevention of cancer includes, but is not limited to, alleviating symptoms associated with cancer, the inhibition of the progression of cancer, the promotion of the regression of cancer, and the promotion of the immune response.

The pharmaceutical compositions of the present invention can be administered alone or in combination with other types of cancer treatment strategies (e.g., radiation therapy, chemotherapy, hormonal therapy, immunotherapy and anti-tumor agents). Examples of anti-tumor agents include, but are not limited to, cisplatin, ifosfamide, paclitaxel, taxanes, topoisomerase I inhibitors (e. g., CPT-11, topotecan, 9-AC, and GG-211), gemcitabine, vinorelbine, oxaliplatin, 5-fluorouracil (5-FU), leucovorin, vinorelbine, temodal, and taxo

Additional embodiments of the present invention are described in the claims and the detailed description of the invention.

### DETAILED DESCRIPTION OF THE INVENTION

### BRIEF DESCRIPTION OF THE FIGURES

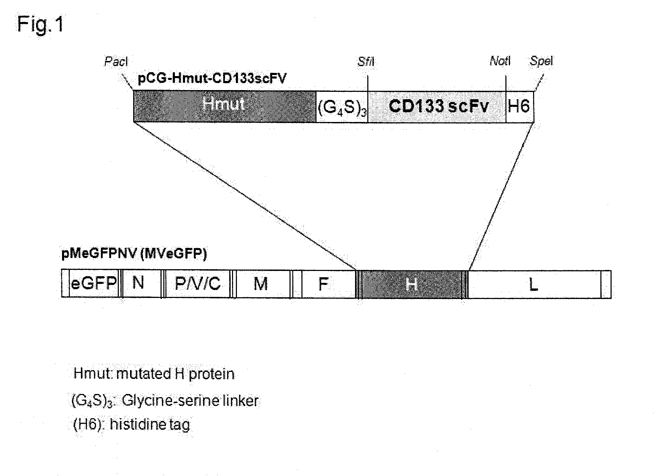

- **Fig. 1:**: Gives an overview over the cloning of MV-CD133, a recombinant tumor stem cell specific and oncolytic measles virus according to the invention.
- **Fig. 2:**: Shows the results of FACS and immuno-staining experiments to verify the tumor stem cell selectivity of MV-CD133.
- **Fig. 3:**: Shows the results of a FACS analysis of CD133 and GFP expression and analysis of the viability of cells infected with MV-CD133.
- **Fig. 4:**: Shows the oncolytic potential of MV-CD133 in immunodeficient (NOD/SCID) mice.
- **Figs. 5/6:**: Show the infection of Glioma tumorshperes by MV-CD133.

### EXAMPLES

Envelope H protein of measles virus was modified to use human CD133 as a receptor for cell entry and to not bind to SLAM and CD46 anymore. The resulting recombinant virus is a tumor stem cell targeting oncolytic virus.

### EXAMPLE 1: Isolation and cloning of CD133 specific scFv and production of recombinant measles virus

For the generation of MV-CD133, RNA prepared from the AC141.7 antibody producing hybridoma HB-12346 generated as described in Yin et al., Blood , 90: 5002-5012.1997 was reverse-transcribed to amplify the IgG variable coding regions of heavy and light chains. A degenerated primer mix (Heavy Primer Mix, #27-1586-01; Light Primer Mix, #27-1583-01; GE Healthcare) was used for reverse transcription-PCR. The resulting PCR fragments were subcloned into the pJETl.2/blunt cloning vector (Fermentas) and then amplified to insert coding sequences for SfiI and NotI restriction sites and the (G₄S)₃-linker using CD133-VH and CD133-VL primer listed in Table 1. The resulting PCR fragments encoding the heavy or light chains were digested with TauI and SfiI, or TauI and NotI, respectively, and inserted by triple ligation into a SfiI and Notl-digested pCG-Hmut backbone resulting in pCG-Hmut-CD133scFv now encoding the cytoplasmic tail-truncated Hmut protein linked to the CD133-specific scFv (Funke et al., Mol.Ther. 16: 1427-1436, 2008; Anliker et al., Nat.Methods 7: 929-935, 2010)

Next, the PacI/SpeI-digested fragment of pCG-Hmut-CD133scFv was inserted into the corresponding sites of pMeGFPNV (MVeGFP), which encodes the full-length infectious clone of the Edmonston lineage measles virus (Duprex et al., J. Virol. 73: 9568-9575, 1999). For the rescue of MV-CD133, the hexahistidine (His6) tagging and retargeting system was used as described previously (Nakamura et al., Nat.Biotechnol. 23: 209-214, 2005). Virus stocks were generated upon infection of Vero-anti-His (anti-His) cells at a multiplicity of infection (MOI) of 0.03, and cell-associated viruses were harvested by multiple freeze-thaw cycles.

**Table 1: CD133-VH and CD133-VL Primer Sequences**

| **Primer** | **Sequence** |
|---|---|
| **CD133-VH forw1** | 5'-GGCCCAGCCGGCCATGGCCCAGGTCCAGCTGCAGGAGTCTGG-3' |
| **CD133-VH rev1** | 5'-GCCGCCACCTCCAGAGCCACCACCTCCCGAGGAGACGGTGACCGTGGTC-3' |
| **CD133-VL forw1** | 5'-GCGGCAGTGGTGGTGGAGGATCCGACATTGTCCTGACCCAGTCTCCA-3' |
| **CD133-VL rev1** | 5'-TGCGGCCGCCCGTTTTATTTCCAGCTTGGTCCC-3' |

### EXAMPLE 2: Verification of tumor stem cell selectivity

HuH7, Vero or HT1080 cells were tested for CD133 expression by flow cytometry applying anti-human CD133/1-PE antibody (clone AC133, Miltenyi). Appropriate isotype controls were used according to the manufacturer's instructions.

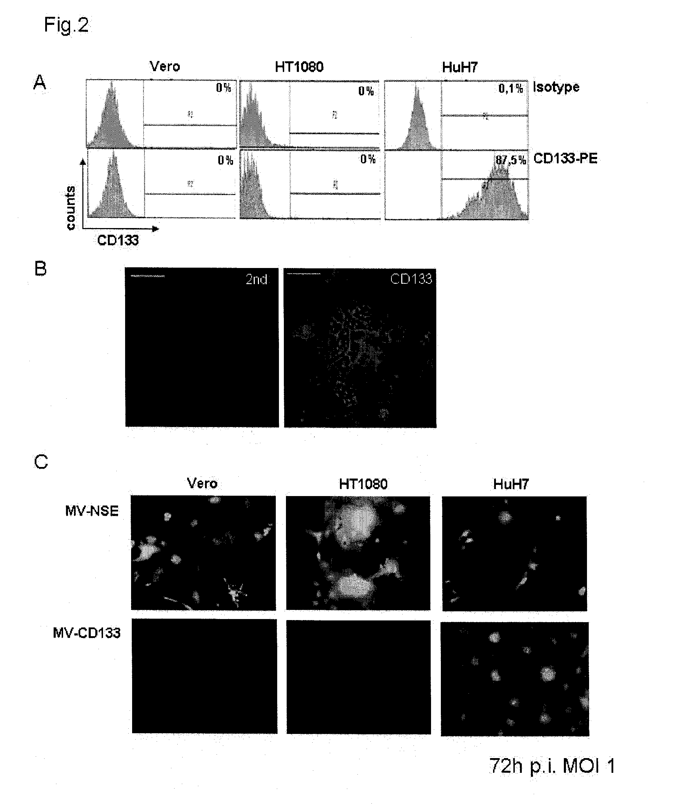

90% of HuH7 cells were CD133 positive whereas HT1080 and Vero cells were CD133 negative (Fig. 2A).

CD133 expression of HuH7 cells was confirmed by immunofluorescence staining using a mouse anti-human CD133 as primary antibody and a donkey anti-mouse IgG Cy3 (Dianova) secondary antibody (Fig. 2B).

HuH7, Vero and HT1080 cells (1×10⁴ cells/24-well plate) were incubated with MV-CD133 or MV-Nse at an MOI of 1 in Opti-MEM at 37 °C. At 72 hours after infection, cells were analysed by fluorescent microscopy. While MV-Nse infected all cell types, MV-CD133 infected only the CD133-positive HuH7 cells. Vero and HT1080 cells that are CD133-negative were not infected with MV-CD133 (Fig. 2C). Thus, MV-CD133 is highly selective for CD133-positive tumor stem cells.

### EXAMPLE 3: Viability of cells infected with MV-CD133

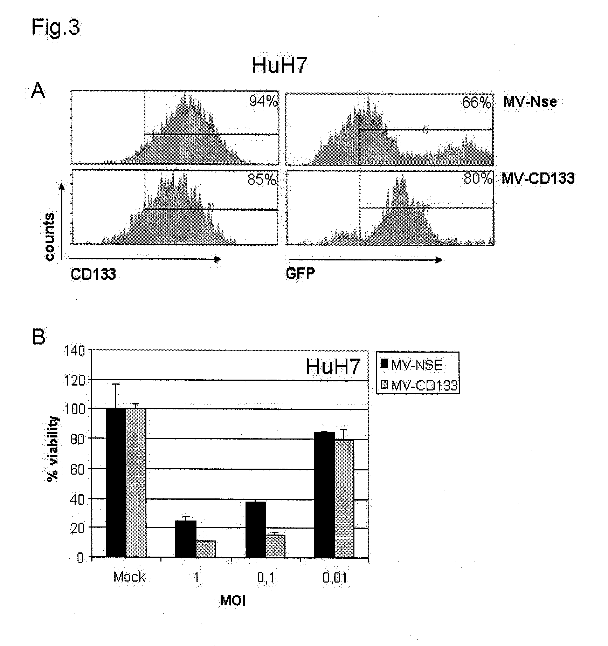

MV-CD133- and MV-Nse-infected HuH7 cells were analyzed for CD133 and GFP expression by FACS analysis. For this purpose, HuH7 cells were infected with an MOI of 1 and two days later the cells were trypsinized and stained for CD133 expression with an anti-human CD133/1-PE antibody and GFP positive cells were analyzed. With MV-CD133 approximately 80% of the cells had become GFP positive, with MV-Nse about 60% of the cells. MV-CD133 infection resulted in slight downregulation of CD133 cell surface expression (Fig. 3A).

To determine the viability of infected cells, the 3-(4,5-dimethylthiazol-2-yl)-2,5-diphenyltetrazolium bromide (Cell Proliferation Kit I (MTT); Roche, Indianapolis, IN) assay was used. Cells were grown in 96-well microtiter plates (1×10⁴ cells/well) in the recommended culture medium.

Infections were at MOI of 0 (mock), 0.01, 0.1, or 1. 96 hours post-infection cell viability was measured by dye absorbence as determined by an optical density measurement at 595 nm on an automated enzyme-linked immunosorbent assay reader. The viability of cells was calculated as the mean of quadruplicate optical density values, divided by the mean of quadruplicate optical density values of identically cultured cells in the absence of virus (which served as control cells) and expressed as a percentage of the control cells.

Compared to mock treated HuH7 cells, MV-CD133 infected HuH7 cells showed only 10% cell viability after infection at an MOI of 1. In case of MV-Nse infected HuH7 cells 20% of the cells were viable. Also at lower MOIs of 0.1 and 0.01 MV-CD133 killed HuH7 cells more efficiently than MV-Nse (Fig. 3B).

### EXAMPLE 4: Oncolytic potential of MV-CD133 in an NOD/SCID mice model.

To determine the oncolytic potential of MV-CD133, HuH7 cells (5 x10⁶ cells in 100 µl) were subcutaneously implanted into the right flank of 6 week old female nonobese diabetic/severe combined immunodeficient (NOD/SCID) mice (Jackson Labs).

After about 10 to 14 days, when the tumors measured 0.5-0.6 cm in diameter, mice received four (one per day) intratumoral injections of 1x10⁶ TCID50 in 100µl Opti-MEM of MV-Nse or MV-CD133 (Clone 141.7)). Control animals (mock therapy groups) were injected with equal volumes of Opti-MEM containing no virus. Tumor growth was then followed over time.

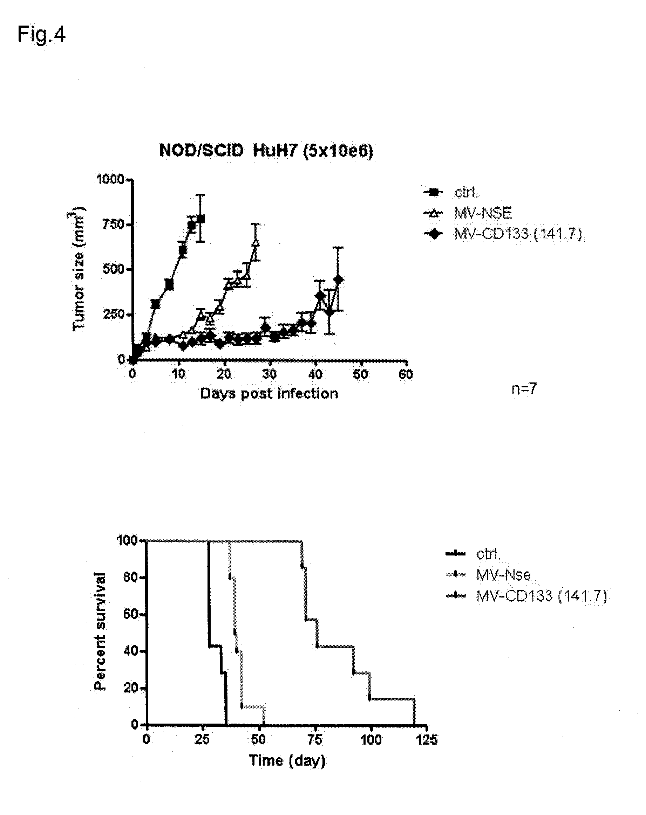

Mice that were treated with MV-CD133 showed a substantial reduction in tumor growth and a significantly prolonged survival period as compared to MV-Nse treated animals or control mice (Fig. 4).

### EXAMPLE 5: Infection of Glioma tumorshperes by MV-CD133

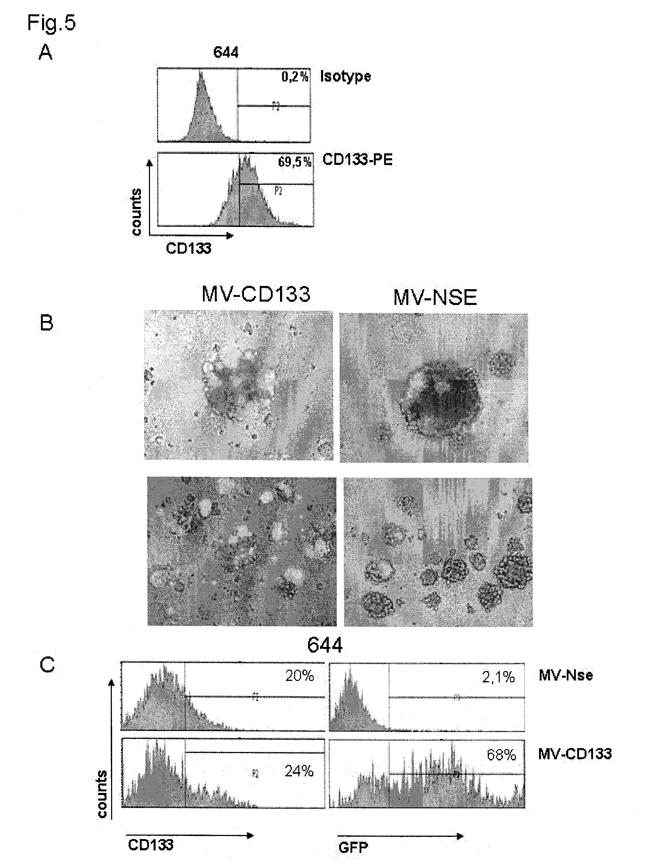

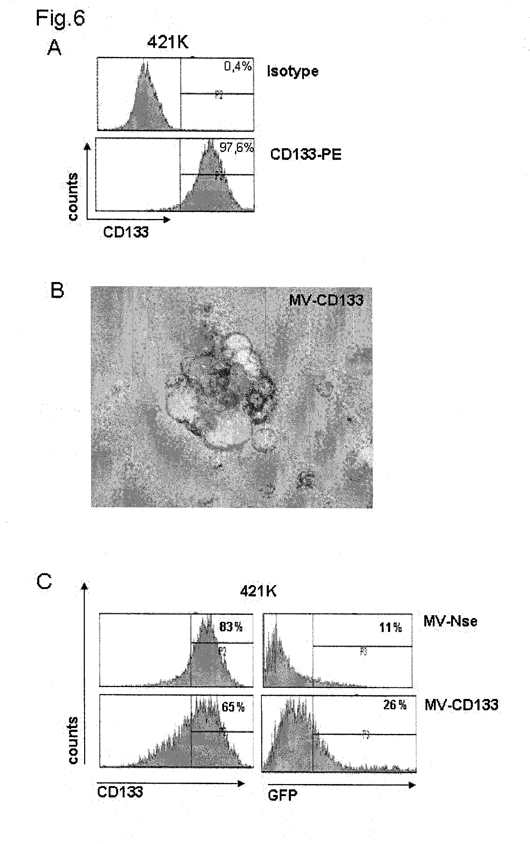

MV-CD133 was tested for infection of Glioma tumorshperes from two different patients (644 or 421K) (Fig. 5 and 6). For this purpose, tumorspheres (1×10⁴ cells/24-well plate) were incubated with viruses (MV-CD133 or MV-Nse) at an MOI of 1 in Opti-MEM at 37 °C. At 48 to 72 hours after infection, cells were photographed by fluorescence microscope.

Both tumorsphere lines were CD133 positive (Fig. 5A and 6A) and were readily infected with MV-CD133 and MV-Nse.

In case of MV-CD133 infection resulted in strong syncitia formation. MV-Nse was also able to infect tumorspheres but to a much reduced level (Fig. 5B and 6B). GFP expression analysis of cells by microscopy correlated well with data obtained by FACS analysis.

Both tumorspheres lines showed high GFP expression levels after MV-CD133 infection and less GFP expression upon infection with MV-Nse (Fig. 5C and 6C).

## Claims

1. An oncolytic virus comprising a recombinant binding domain specific for a tumor stem cell marker.

2. The oncolytic virus according to claim 1, wherein the virus has a decreased specificity for its original receptor(s) used for cell entry.

3. The oncolytic virus according to any of the preceding claims, wherein the tumor stem cell marker is CD133.

4. The oncolytic virus according to any of the preceding claims, wherein the recombinant binding domain comprises a single-chain variable fragment (scFv).

5. The oncolytic virus according to claim 4, wherein the scFv is derived from hybridoma cell line HB-12346.

6. The oncolytic virus according to any of the preceding claims further comprising a suicide gene.

7. The oncolytic virus according to any of the preceding claims, wherein the virus is from the Paramyxoviridae family, genus Morbillivirus, preferably a measles virus (MeV).

8. The oncolytic virus accoridng to any of the preceeding claims as a medicament.

9. The oncolytic virus according to claim 8 for the treatment or prevention of cancer, glioma, liver carcinoma and/or colon carcinoma.

10. The oncolytic virus according to claim 9 to be used in combination with other types of cancer treatment strategies.


SEQUENCE LISTING

<110>  Bundesrepublik Deutschland, letztvertreten durch den
       Präsidenten des Paul-Ehrlich-Instituts Prof. Dr. Klaus Cichutek

<120>  Enhanced tumor therapy by tumor stem cell targeted oncolytic
       viruses

<130>  141-007

<160>  4

<170>  PatentIn version 3.5

<210>  1
<211>  42
<212>  DNA
<213>  Artificial Sequence

<220>
<223>  Forward Primer

<400>  1
ggcccagccg gccatggccc aggtccagct gcaggagtct gg
42


<210>  2
<211>  49
<212>  DNA
<213>  Artificial DNA sequence

<220>
<223>  Reverse primer

<400>  2
gccgccacct ccagagccac cacctcccga ggagacggtg accgtggtc
49


<210>  3
<211>  47
<212>  DNA
<213>  Artificial DNA sequence

<220>
<223>  Forward primer

<400>  3
gcggcagtgg tggtggagga tccgacattg tcctgaccca gtctcca
47


<210>  4
<211>  33
<212>  DNA
<213>  Artificial DNA sequence

<220>
<223>  Reverse primer

<400> 4
tgcggccgcc cgttttattt ccagcttggt ccc
33